(19) Europäisches Patentamt
European Patent Office
Office européen des brevets

(11) **EP 3 492 577 A1**

(12) **EUROPEAN PATENT APPLICATION**
published in accordance with Art. 153(4) EPC

(43) Date of publication:
**05.06.2019 Bulletin 2019/23**

(51) Int Cl.:
*C12M 3/00* *(2006.01)*          *C12M 1/00* *(2006.01)*
*C12N 5/071* *(2010.01)*        *C12N 5/0735* *(2010.01)*
*C12N 5/10* *(2006.01)*         *C12N 5/074* *(2010.01)*

(21) Application number: **17834568.2**

(22) Date of filing: **28.07.2017**

(86) International application number:
**PCT/JP2017/027557**

(87) International publication number:
**WO 2018/021566 (01.02.2018 Gazette 2018/05)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **29.07.2016 JP 2016150725**

(71) Applicants:
• **Daikin Industries, Ltd.**
  **Osaka-shi, Osaka 530-8323 (JP)**
• **Kyoto University**
  **Sakyo-ku**
  **Kyoto-shi**
  **Kyoto 606-8501 (JP)**

(72) Inventors:
• **KOMAZAWA, Kozue**
  **Osaka-shi**
  **Osaka 530-8323 (JP)**
• **HIGUCHI, Tatsuya**
  **Osaka-shi**
  **Osaka 530-8323 (JP)**
• **KANEKO, Shin**
  **Kyoto-shi**
  **Kyoto 606-8501 (JP)**
• **YASUI, Yutaka**
  **Kyoto-shi**
  **Kyoto 606-8501 (JP)**

(74) Representative: **Hoffmann Eitle**
**Patent- und Rechtsanwälte PartmbB**
**Arabellastraße 30**
**81925 München (DE)**

(54) **DIFFERENTIATED CELL PRODUCTION METHOD AND CULTURE BAG USED THEREFOR**

(57)    The present invention addresses the problem of providing a method that enables an embryoid body (EB) method to be carried out on a large scale in a closed system, as compared with conventional methods using a culture dish. The present invention provides a method for producing differentiated cells from pluripotent stem cells by an EB method, comprising the steps of: (1) culturing pluripotent stem cells by using a culture bag having a perfluoropolymer on its inner surface to thereby form an embryoid body; and (2) inducing differentiation of the pluripotent stem cells contained in the embryoid body obtained in step (1) to thereby obtain differentiated cells.

Fig. 1

**Description**

Technical Field

The present invention relates to a method for producing differentiated cells and to a culture bag for use in the production method.

Background Art

Pluripotent stem cells, which have the ability to differentiate into various cells, are expected to find clinical application or application to drug discovery research etc. Studies on using induced pluripotent stem cells (iPS cells) and embryonic stem cells (ES cells) as pluripotent stem cells are ongoing.
In use of pluripotent stem cells, a method for efficiently inducing differentiation into a desired differentiated cell is required. A method frequently used to induce differentiation of pluripotent stem cells is the "embryoid body (EB) method," which comprises culturing in a low-adherent culture dish (Petri dish) to allow for spontaneous aggregation of pluripotent stem cells, thereby forming an embryoid body (EB), which is a three-dimensional mass. The efficiency of induction into a desired cell species can be enhanced by adding cytokine, a growth factor, or other compounds that have differentiation-inducing effects on culture of an embryoid body.
In the EB method, for example, a low-adherenct Petri dish coated with a hydrogel or a hydrophilic polymer that has a phospholipid analogous structure, such as 2-methacryloyloxyethyl phosphorylcholine, has been used as a non-adherent culture dish (Petri dish) (PTL 1).

Citation List

Patent Literature

PTL 1: WO2005/001019

Summary of Invention

Technical Problem

An object of the present invention is to provide a method that enables an EB method to be carried out on a large scale in a closed system, as compared with conventional methods using a culture dish.

Solution to Problem

To achieve the above object, the present inventors carried out extensive research and arrived at the idea that bag culture, which enables a larger amount of pluripotent stem cells to be cultured in a suspension, would be advantageous over conventional culture using Petri dishes. However, the inventors found it difficult to produce a gas-permeable culture bag by using the same material as that of Petri dishes conventionally used for the EB method. The inventors further repeated trial and error and found that by using a culture bag having a sufficiently gas-permeable and superhydrophobic perfluoropolymer on its inner surface, the EB method can be carried out in a closed system on a larger scale than, and as efficiently as, conventional methods using a culture dish. The inventors thus achieved the present invention.
The present invention has been accomplished based on the above findings, and includes the following embodiments.

Item 1. A method for producing differentiated cells from pluripotent stem cells by an embryoid body (EB) method, comprising the steps of:

(1) culturing pluripotent stem cells by using a culture bag having a perfluoropolymer on its inner surface to thereby form an embryoid body; and
(2) inducing differentiation of the pluripotent stem cells contained in the embryoid body obtained in step (1) to thereby obtain differentiated cells.

Item 2.
A method for inducing differentiation of pluripotent stem cells by an embryoid body (EB) method, comprising the steps of:

(1) culturing pluripotent stem cells by using a culture bag having a perfluoropolymer on its inner surface to thereby form an embryoid body; and
(2) inducing differentiation of the pluripotent stem cells contained in the embryoid body obtained in step (1) to thereby obtain differentiated cells.

Item 3.
The method according to Item 1 or 2, wherein the perfluoropolymer is at least one perfluoropolymer selected from the group consisting of tetrafluoroethylene-hexafluoropropylene copolymers, tetrafluoroethylene-perfluoroalkyl vinyl ether copolymers, and tetrafluoroethylene-hexafluoropropylene-perfluoroalkyl vinyl ether copolymers.
Item 4.
The method according to any one of Items 1 to 3, wherein the pluripotent stem cells are induced pluripotent stem cells (iPS cells) or embryonic stem cells (ES cells).
Item 5.
The method according to any one of Items 1 to 4, wherein in step (2), the pluripotent stem cells are differentiated into progenitor cells.
Item 6.
A culture bag having a perfluoropolymer on its inner surface, the bag being for use in inducing differentiation of pluripotent stem cells by an embryoid body (EB) method.
Item 7.
The culture bag according to Item 6, wherein the perfluoropolymer is at least one perfluoropolymer selected from the group consisting of tetrafluoroethylene-hexafluoropropylene copolymers, tetrafluoroethylene-perfluoroalkyl vinyl ether copolymers, and tetrafluoroethylene-hexafluoropropylene-perfluoroalkyl vinyl ether copolymers.
Item 8.
The culture bag according to Item 6 or 7, wherein the pluripotent stem cells are induced pluripotent stem cells (iPS cells) or embryonic stem cells (ES cells).
Item 9.
An embryoid body that is obtainable by step (1) according to any one of Items 1 to 4.
Item 10.
A differentiated cell population that is obtainable by the production method according to any one of Items 1 and 3 to 5.

Advantageous Effects of Invention

According to the present invention, an EB method can be carried out on a larger scale than conventional methods using a culture dish.

Brief Description of Drawings

Fig. 1 illustrates an example of the culture bag of the present invention.
Fig. 2 shows results of the Example.
Fig. 3 shows results of the Example.
Fig. 4 shows results of the Example.
Fig. 5 shows results of the Example.
Fig. 6 shows results of the Example.
Fig. 7 shows results of the Example.
Fig. 8 shows results of the Example.
Fig. 9 shows results of the Example.
Fig. 10 shows results of the Example.
Fig. 11 shows results of the Example.
Fig. 12 shows results of the Example.
Fig. 13 shows results of the Example.
Fig. 14 shows results of the Example.
Fig. 15 shows results of the Example.
Fig. 16 shows results of the Example.
Fig. 17 shows results of the Example.
Fig. 18 shows results of the Example.
Fig. 19 shows results of the Example.

Fig. 20 shows results of the Example.
Fig. 21 shows results of the Example.
Fig. 22 shows results of the Example.

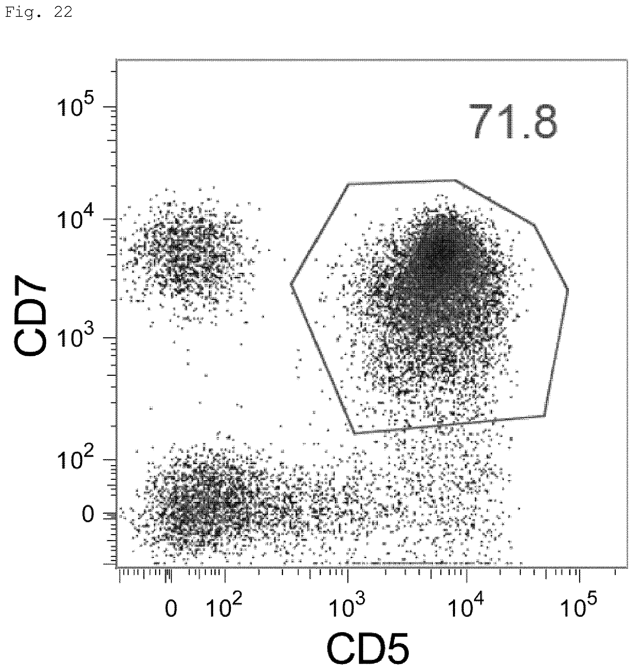

Fig. 22 shows results of the Example.

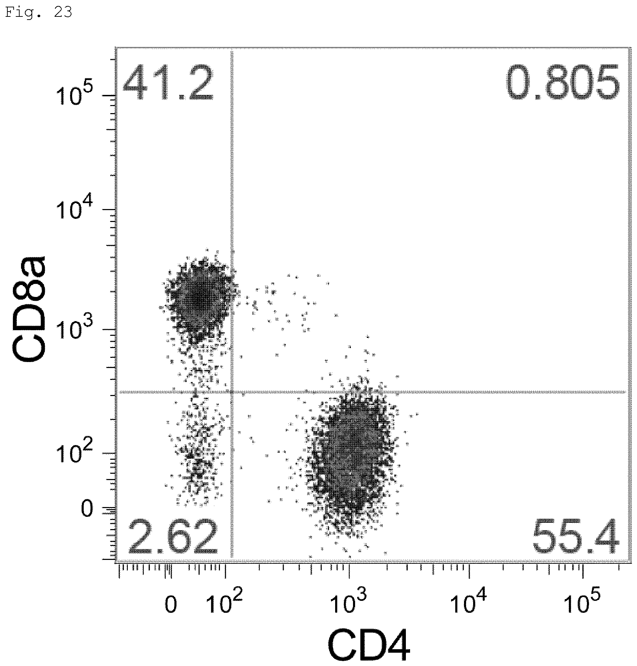

Fig. 23 shows results of the Example.

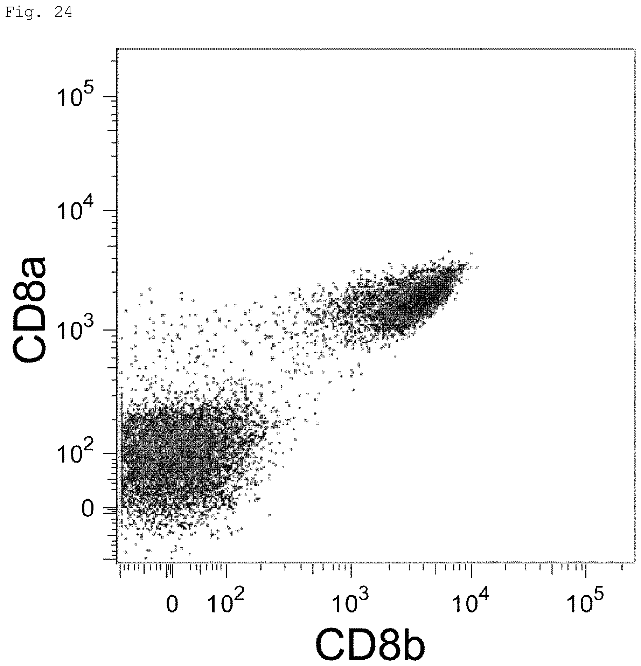

Fig. 24 shows results of the Example.

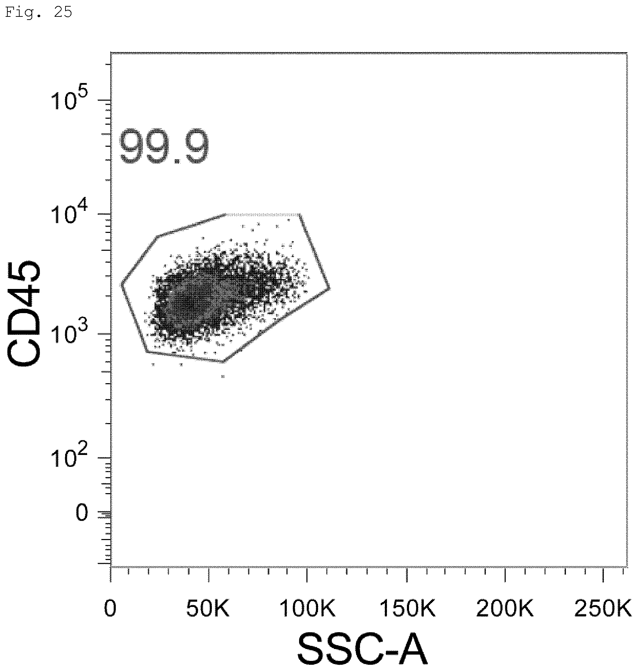

Fig. 25 shows results of the Example.

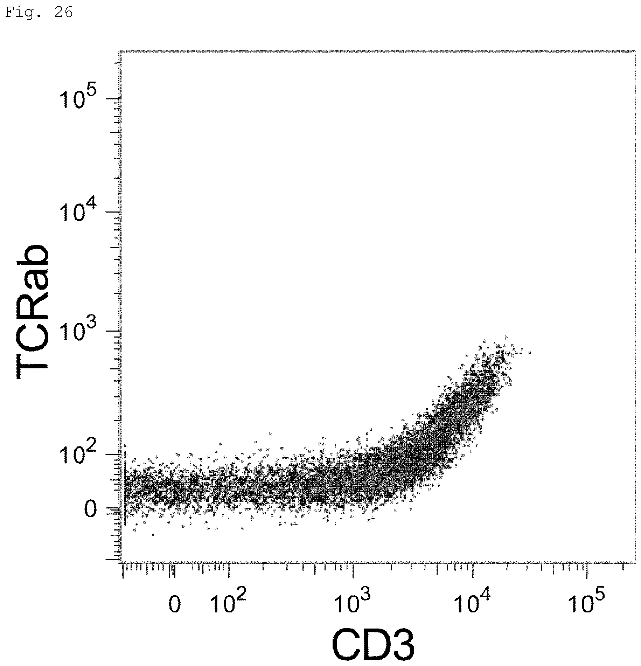

Fig. 26 shows results of the Example.

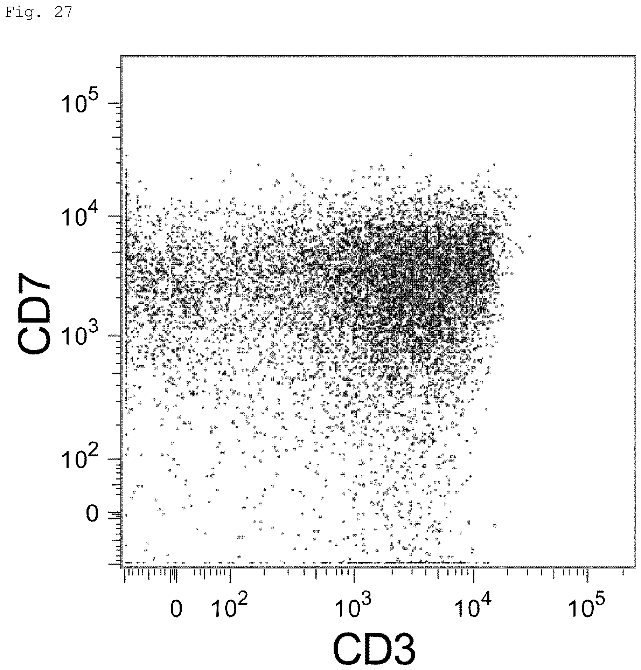

Fig. 27 shows results of the Example.

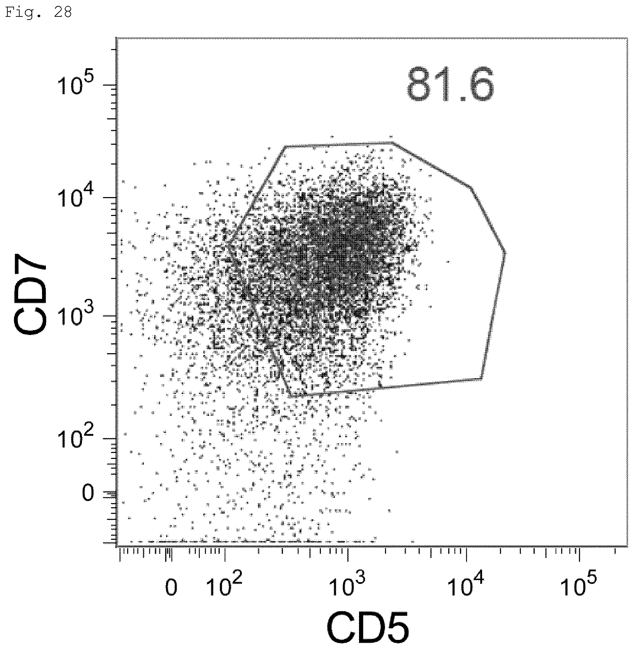

Fig. 28 shows results of the Example.

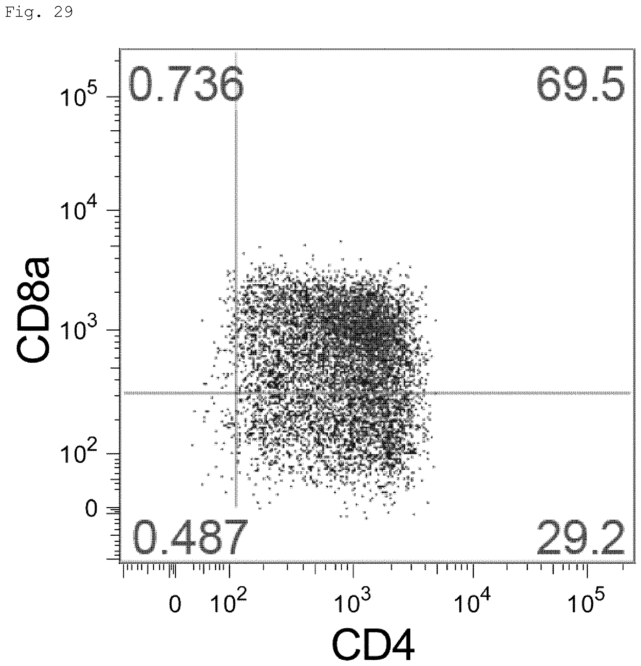

Fig. 29 shows results of the Example.

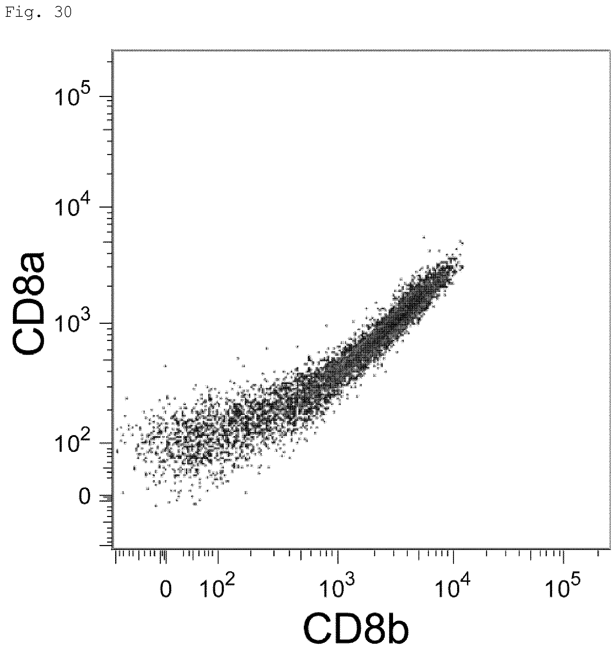

Fig. 30 shows results of the Example.

Description of Embodiments

1. Method for Producing Differentiated Cells and Culture Bag for Use in the Method

The method for producing differentiated cells of the present invention is a method for producing differentiated cells from pluripotent stem cells by an embryoid body (EB) method, the method comprising the steps of:

(1) culturing pluripotent stem cells by using a culture bag having a perfluoropolymer on its inner surface to thereby form an embryoid body; and
(2) inducing differentiation of the pluripotent stem cells contained in the embryoid body obtained in step (1).

1.1 Step (1)

1.1.1 Culture Bag

The culture bag comprises a perfluoropolymer on its inner surface. The surface with which the culture cells may come into contact during the culture is covered with at least a perfluoropolymer. This prevents pluripotent stem cells from adhering to the surface of the culture bag, whereby an embryoid body can be formed normally.

The culture bag may be formed of a sufficiently gas-permeable perfluoropolymer itself. In that case, the film that forms the culture bag is composed of a perfluoropolymer itself and may optionally further have one or more coatings, if necessary.

To obtain the effect of the present invention, the perfluoropolymer must be present in the portion with which cells come into contact. As long as this condition is met, such coatings can be optionally applied. Accordingly, except for the case in which there are special circumstances, such coatings, if used, are usually applied to the outside of the culture bag or portions with which cells do not come into contact.

The size of the culture bag is not limited and can be suitably set. The lower limit of the capacity of the culture bag may be, for example, but is not limited to, 10 ml, 20 ml, 50 ml, or 100 ml. The upper limit of the capacity may be, for example, but is not limited to, 500 ml, 400 ml, 300 ml, or 200 ml. The capacity range of the culture bag may be, for example, 10 ml to 500 ml.

In this specification, "perfluoropolymer" refers to a polymer that basically does not contain hydrogen in the molecule and that is composed of carbon and fluorine, or a polymer that is basically composed of carbon and fluorine and that partially contains oxygen etc.

The perfluoropolymer optionally contains a non-fluorinated group terminal. In the present invention, "non-fluorinated group terminal" refers to an end that is reactive and is generally called an "unstable end." Specific examples include functional groups, such as -COF, -COOH, -COOH associated with water, $-CH_2OH$, $-CONH_2$ and $-COOCH_3$. Such non-fluorinated group terminals are formed by being derived from, for example, a reaction initiator in a polymerization reaction.

The perfluoropolymer is preferably a perfluoropolymer in which the total number of non-fluorinated group terminals is 70 or less per $1 \times 10^6$ carbon atoms. In this case, high non-adherence to cells can be exhibited. In a more specific embodiment of the present invention, the perfluoropolymer is preferably a perfluoropolymer in which the total number of -COF, -COOH, -COOH associated with water, $-CH_2OH$, $-CONH_2$ and $-COOCH_3$ is preferably 70 or less per $1 \times 10^6$ carbon atoms.

The total number of the non-fluorinated group terminals per $1 \times 10^6$ carbon atoms is preferably, with increasing preference in the order given, 50 or less, 35 or less, 15 or less, 10 or less, 5 or less, or 2 or less. The same applies to the total number of -COF, -COOH, -COOH associated with water, $-CH_2OH$, $-CONH_2$ and $-COOCH_3$.

The perfluoropolymer is preferably a perfluoropolymer in which the total number of non-fluorinated group terminals and -CF$_2$H group terminals is 70 or less per 1 x 10$^6$ carbon atoms. The total number of the non-fluorinated group terminals and -CF$_2$H group terminals per 1 x 10$^6$ carbon atoms is preferably, with increasing preference in the order given, 35 or less, 20 or less, or 10 or less.

Preferably, the perfluoropolymer does not contain -CF$_2$H group terminals.

The number of the non-fluorinated group terminals and -CF$_2$H group terminals can be calculated by FT-IR.

The method for adjusting the amount of non-fluorinated groups in the perfluoropolymer to the range mentioned above may be, for example, but is not limited to, the following methods: a method of controlling terminal groups by using a chain transfer agent or a polymerization catalyst in a polymerization reaction to thereby inhibit creation of non-fluorinated group terminals; a method of fluorinating non-fluorinated group terminals by bringing a polymer obtained by a polymerization reaction into contact with a fluorine-containing compound, which functions as a fluorine radical source; and the like.

In the above, the operation of bringing the perfluoropolymer obtained by the polymerization reaction into contact with a fluorine-containing compound, which functions as a fluorine radical source, can also be performed at any stage before or after melt extrusion after a perfluoropolymer is obtained by a method such as suspension polymerization or emulsion polymerization. This operation can also be performed at a stage after shaping a perfluoropolymer. This operation can be effectively performed by repeating the operation at two or more of the following three stages: before and after melt extrusion, and after shaping.

The fluorine-containing compound that functions as a fluorine radical source and that is used in the method of fluorinating non-fluorinated group terminals is not particularly limited and can be selected from a wide variety of fluorine-containing compounds. Examples include fluorinated halogens, such as IFs and ClF$_3$, fluorine gas (F$_2$), CoF$_3$, AgF$_2$, UF$_6$, OF$_2$, N$_2$F$_2$, CF$_3$OF, and the like.

When fluorine gas is used, 100% fluorine gas may be used. However, mixing fluorine gas with an inert gas and then using the mixture is recommendable in terms of safety. In that case, the mixing ratio may be, for example, but is not limited to, a fluorine gas concentration of 5 to 50 mass%. A fluorine gas concentration of 15 to 30 mass% is particularly preferable. The inert gas to be used is not limited, and may be selected from a wide variety of gases, such as nitrogen gas, helium gas, and argon gas. Nitrogen gas is preferable in terms of cost performance.

In the method of fluorinating non-fluorinated group terminals comprising bringing the polymer obtained by a polymerization reaction into contact with a fluorine-containing compound, which functions as a fluorine radical source, the processing temperature is preferably 20 to 220°C, and more preferably 100 to 200°C. The processing time is preferably 5 to 30 hours, and more preferably 10 to 20 hours.

The perfluoropolymer is preferably a homopolymer or a copolymer comprising repeating units derived from at least one perfluoroethylenic monomer.

The perfluoropolymer preferably comprises repeating units derived from at least one fluoroethylenic monomer selected from the group consisting of tetrafluoroethylene (TFE), hexafluoropropylene (HFP), and perfluoro(alkyl vinyl ether) (PAVE) represented by CF$_2$=CF-ORf (wherein Rf is a perfluoroalkyl group having 1 to 8 carbon atoms).

Examples of PAVE include, but are not limited to, perfluoro(methyl vinyl ether) (PMVE), perfluoro(ethyl vinyl ether) (PEVE), perfluoro(propyl vinyl ether) (PPVE), perfluoro(butyl vinyl ether), perfluoro(pentyl vinyl ether), perfluoro(hexyl vinyl ether), perfluoro(heptyl vinyl ether), and the like.

Examples of perfluoropolymers include polytetrafluoroethylenes (PTFE), TFE-HFP copolymers (FEP), TFE-PAVE copolymers (PFA), TFE-HFP-PAVE copolymers, and the like. Among these, FEP and PFA are preferable, and FEP is more preferable.

The mass ratio of TFE:HFP in FEP is preferably in the range of 80:20 to 97:3, and more preferably 84:16 to 92:8.

The mass ratio of TFE:PAVE in PFA is preferably in the range of 90:10 to 98:2, and more preferably 92:8 to 97:3.

The mass ratio of TFE:HFP:PAVE in the TFE-HFP-PAVE copolymer is preferably 70 to 97:2.5 to 20:0.1 to 10.

The culture bag can be produced, for example, by superposing two sheets of film comprising a perfluoropolymer as described above and then heat-sealing the edge portions by laser joining or using an impulse sealer, or the like.


1.1.2 Pluripotent Stem Cells


The pluripotent stem cells to be used are not limited and may be, for example, undifferentiated cells that have both self-replication ability and differentiation pluripotency. Self-replication ability means that cells can proliferate while maintaining an undifferentiated state. Differentiation pluripotency means that cells can differentiate into all three germ layer lineages.

Specific examples of pluripotent stem cells include ES cells, iPS cells, embryonal carcinoma (EC) cells, which are derived from teratocarcinoma cells, embryonic germ (EG) cells, which are derived from primordial germ cells, multipotent germline stem (mGS) cells, which can be isolated during the process of establishing and culturing GS cells from testicular tissue, and multipotent adult progenitor cells (MAPC), which can be isolated from bone marrow.

Examples of usable ES cells include ES cells produced by nuclear reprogramming of somatic cells.

Examples of pluripotent stem cells that can be preferably used include ES cells and iPS cells.

The origin of pluripotent stem cells is not limited, and can be selected from mammals according to the purpose of use of the desired differentiated cells. As long as pluripotent stem cells can be established, the origin of the cells can be selected, for example, from humans, monkeys, pigs, rabbits, dogs, rats, mice, etc.

The origin of iPS cells is not particularly limited, and can be appropriately selected from various somatic cells. Examples include fibroblasts, synoviocytes, T lymphocytes, dental pulp stem cells, umbilical cord blood cells, peripheral mononuclear blood cells, and the like.

The concentration of pluripotent stem cells in the culture is not limited, and can be appropriately set according to, for example, the kind of cells used. The concentration may be, for example, $3.3 \times 10^3$ to $3.3 \times 10^5$ cells/ml, preferably $3.3 \times 10^4$ to $3.3 \times 10^5$ cells/ml, and more preferably about $3.3 \times 10^4$ cells/ml.

### 1.1.3 Culture Conditions

The culture conditions are not particularly limited, and can follow the conditions used in a usual EB method. The culture conditions may be optionally modified appropriately.

The process up to the formation of an embryoid body can be summarized as follows. Pluripotent stem cells are dispersed in a culture liquid, which may contain various additives, such as serum and a growth factor, if necessary, to thereby obtain a cell suspension. The cell suspension is placed in the culture container of the present invention and cultured in a predetermined environment, such as in a $CO_2$ incubator. An embryoid body is usually formed in about 2 to 7 days after initiating the culture.

The culture is preferably performed while shaking the culture bag. The shaking conditions are not limited, and can be appropriately set.

### 1.2 Step (2)

Step (2) is a step of obtaining differentiated cells by inducing differentiation of pluripotent stem cells contained in the embryoid body obtained in step (1) described above.

The conditions for inducing the differentiation are not limited, and can be appropriately set according to, for example, the kind of cell used and the kind of desired differentiated cell. The differentiation can be usually induced by adding a specific cytokine, growth factor, or other compounds to a culture liquid in predetermined concentrations, and culturing.

Steps (1) and (2) may be performed simultaneously. In this case, step (1) can be performed in the presence of the compound used in step (2).

The differentiated cells are not limited, and can be any of various progenitor cells. For example, hematopoietic progenitor cells can be obtained by using T lymphocyte-derived T-iPS cells as pluripotent stem cells and performing steps (1) and (2) under predetermined conditions.

### 2. Embryoid Body and Differentiated Cell Population

The embryoid body of the present invention can be obtained by step (1) in the production method of the present invention.

The embryoid body of the present invention is considered to have at least properties similar to those of an embryoid body obtained by the conventional EB method.

The differentiated cell population is a population of differentiated cells obtained by the production method of the present invention.

### Examples

Experimental Examples (including Examples and Comparative Examples) are given below to further clarify the structural features and effects of the present invention. The Experimental Examples are merely examples to facilitate understanding of the present invention. The scope of the invention is not limited by the Experimental Examples.

### Examples 1 to 4: Production of Culture Bags

Pellets of FEP and PFA, individually used as materials, were melted and shaped to thereby form different types of film.

Two types of film were prepared in the same manner as above except that the pellets were fluorinated when melt-extruded.

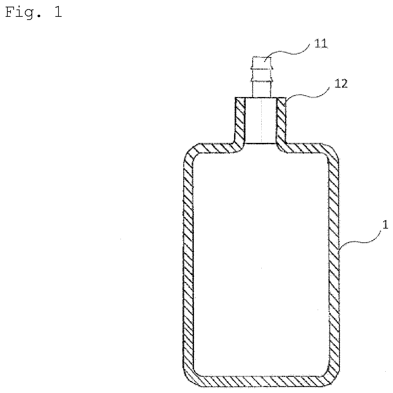

Each type of film with a size of 106 mm x 66 mm and a thickness of 100 $\mu$m was heat-sealed using an impulse sealer under the following conditions: with a seal width of 3 mm, at a sealing pressure of 0.2 MPa, for a sealing time of 50 seconds. One port made of the same material as each film was attached to an upper portion of the heat-sealed film to thereby form four kinds of bags (Examples 1 to 4) (Fig. 1).

A sample of each material with a thickness of about 250 to 300 $\mu$m was formed by superposing the same type of film on one another. The samples thus obtained were analyzed with an FT-IR Spectrometer 1760X (produced by Perkin-Elmer Inc.).

The samples were prepared by superposing the same type of film.

A differential spectrum between a reference (a sample fluorinated enough until substantially no difference in spectra was able to be seen) and each sample was obtained. Absorbance of each peak was read and the number of non-fluorinated group terminals and the number of -CF$_2$H group terminals per $1 \times 10^6$ carbon atoms were calculated according to the following equation. Table 2 shows the number of non-fluorinated group terminals and the number of -CF$_2$H group terminals in each culture bag.

$$\texttt{The total number of non-fluorinated group terminals and -CF}_2\texttt{H}$$
$$\texttt{group terminals (per } 1 \times 10^6 \texttt{ carbon atoms) = I} \cdot \texttt{k/t}$$

I: Absorbance
K: Correction coefficient (Table 1)
t: Thickness of film (mm)

Table 1

| Terminal group | Absorption wavenumber (cm$^{-1}$) | Correction coefficient |
|---|---|---|
| -COF | 1884 | 405 |
| -COOH (not associated with water) | 1813 | 455 |
| =COOH (associated with water) | 1775<br>1790 | 455 |
| -COOCH$_3$ | 1795 | 355 |
| -CONH$_2$ | 3438 | 480 |
| -CH$_2$OH | 3648 | 2325 |
| -CH$_2$H | 3006 | 26485 |

[Table 2]

| | Fluorine resin | Number of non-fluorinated group terminals | Number of -CF$_2$H group terminals |
|---|---|---|---|
| Example 1 | FEP | 21 | 424 |
| Example 2 | FEP | 13 | 0 |
| Example 3 | PFA | 201 | 159 |
| Example 4 | PFA | 25 | 0 |

Example 5: Production of Differentiation-induced Cell Population

(1) Preparation of EB

T-iPS cells were cultured on a plastic plate (BD Biosciences) coated with Matrigel (Corning Life Sciences).

Cultured undifferentiated T-iPS cells were treated with TrypLE (GIBCO), and the cells were gently dissociated. The T-iPS cells were obtained by the method disclosed in a known publication (Nishimura et al., "Generation of Rejuvenated Antigen-Specific T Cells by Reprogramming to Pluripotency and Redifferentiation," Cell Stem Cell, 2013, 12, pp. 114-126).

A cell aggregate was resuspended in StemPro-34 (Invitrogen) containing penicillin-streptomycin (10 ng/mL), L-glutamine (2 mM), ascorbic acid (1 mM), monothioglycerol (MTG, 4 x 10-4 M; Sigma), transferrin (150 μ/mL), and BMP-4 (osteogenic protein-4) (10 ng/mL).

15 ml of the cell suspension was placed in each of the bags obtained in Examples 1 to 4 and cultured. The number of cells on initiation of the culture was set to $5 \times 10^{5}$-/bag and $5 \times 10^{6}$-/bag.

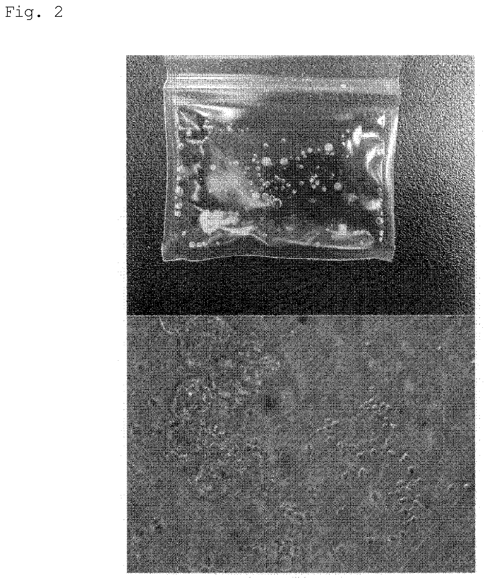

After 24 hours, bFGF (a basic fibroblast growth (proliferation) factor) was added to a final concentration of 5 ng/ml, and the culture was continued. As a result, formation of EB was observed in all of the bags (Fig. 2).

On day 14 after initiation of the culture, the cells were collected, and differentiation tendency was analyzed using FACS. FACSAria™II, produced by BD Biosciences, was used as a flow cytometer.

For this analysis, fluorescently labeled antibodies against CD34 (fluorescently labeled with APC), CD34 (fluorescently labeled with Pacific Blue), CD43 (fluorescently labeled with PE), CD-14 (fluorescently labeled with PE-Cy7), and CD235a (fluorescently labeled with APC) were used.

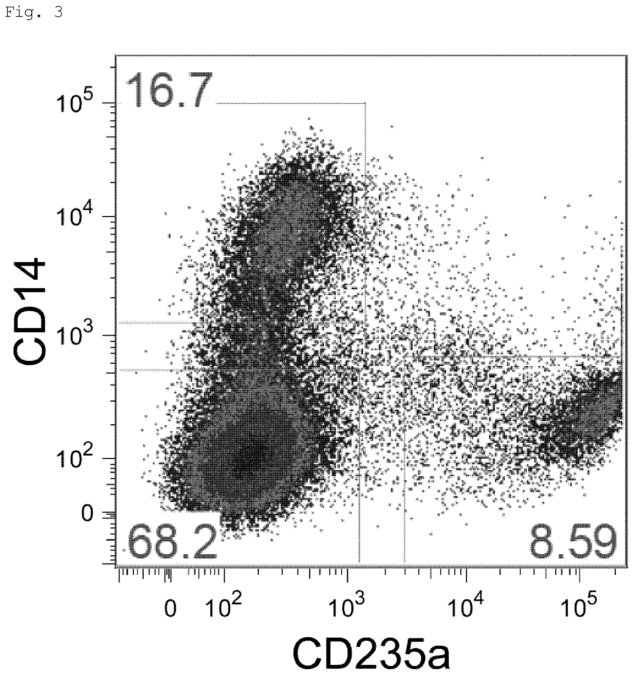

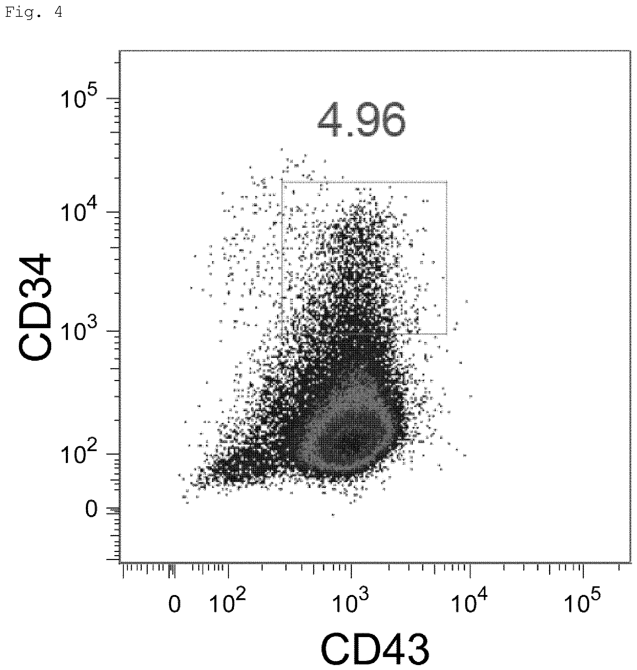

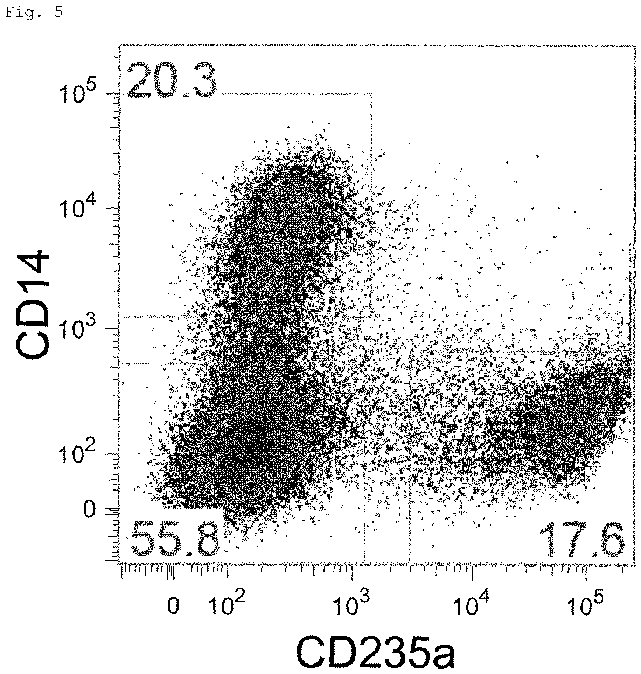

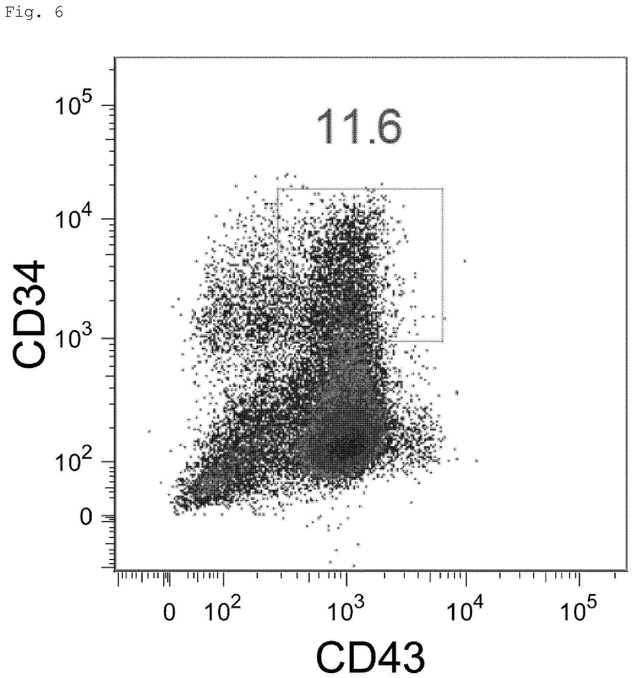

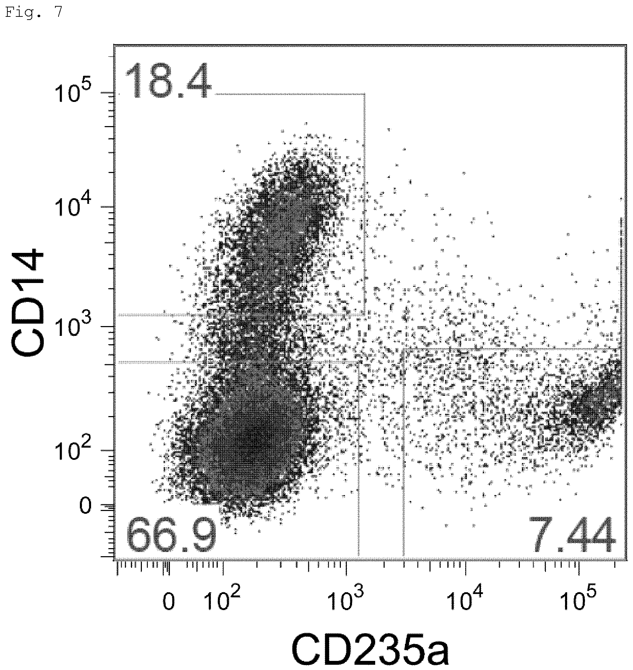

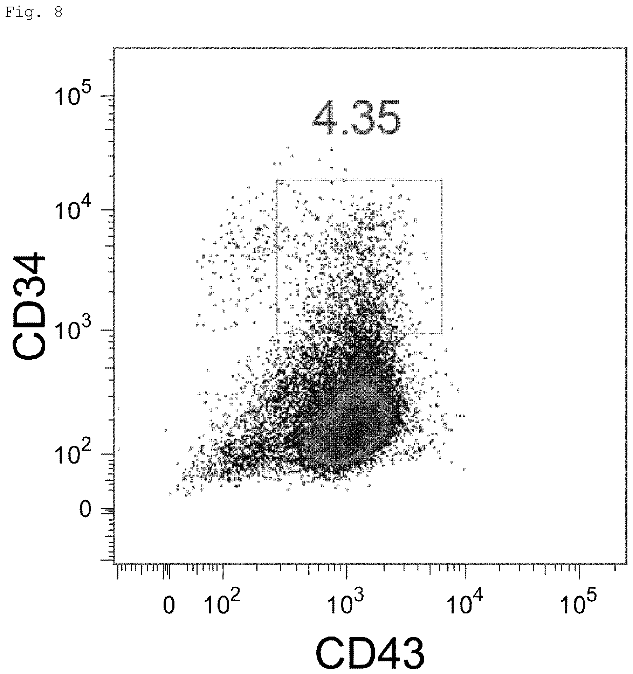

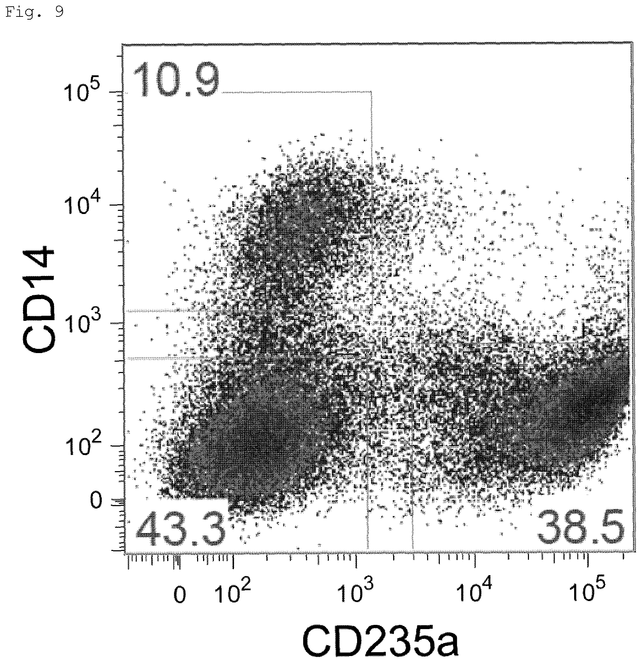

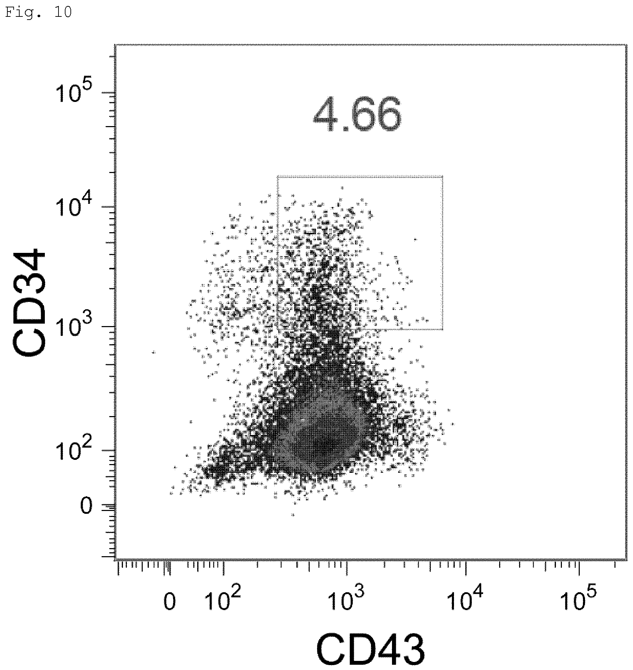

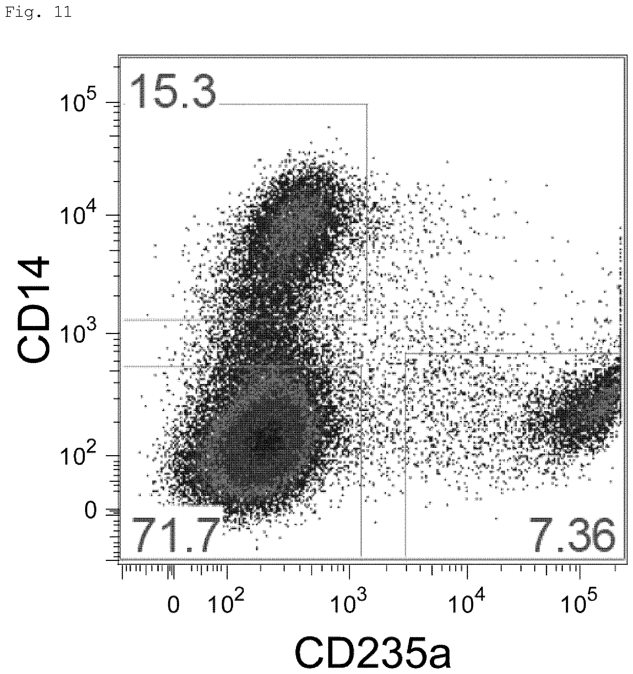

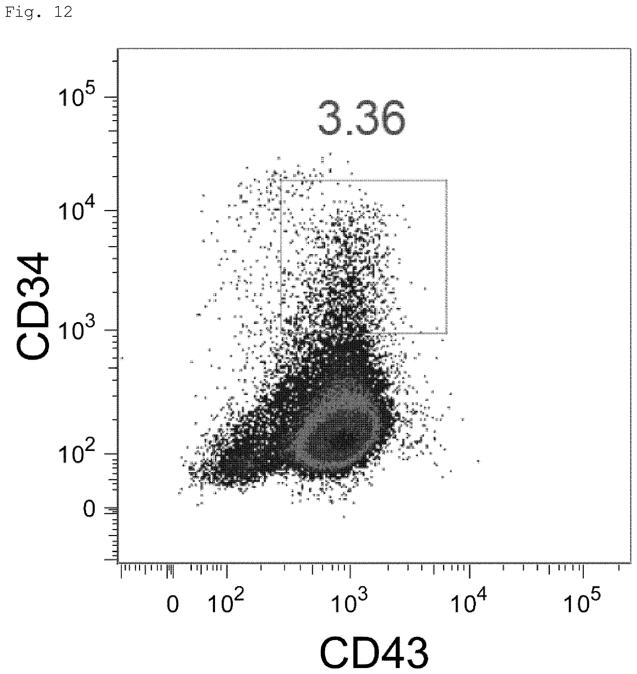

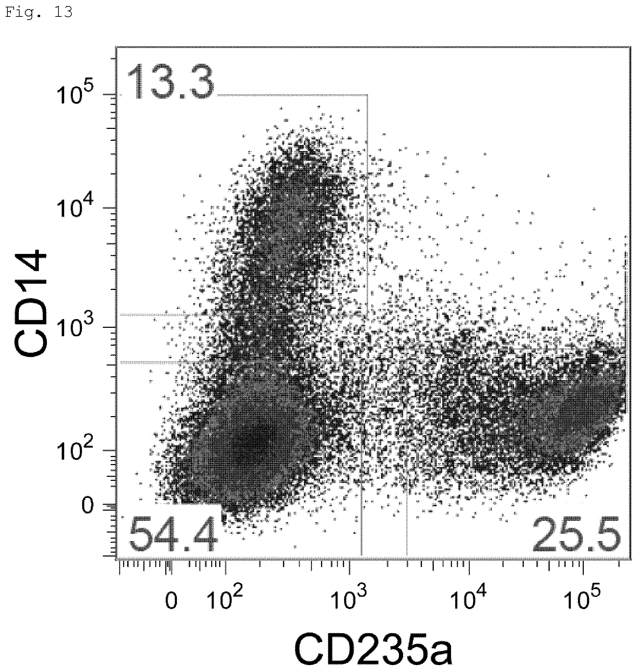

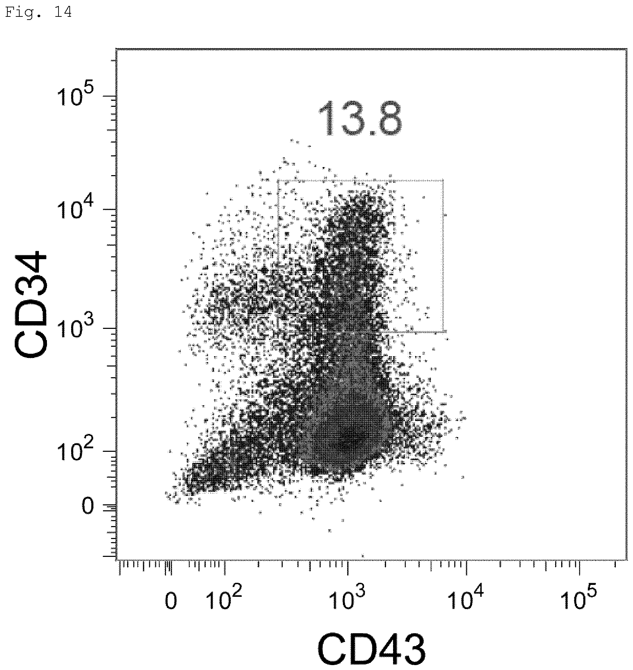

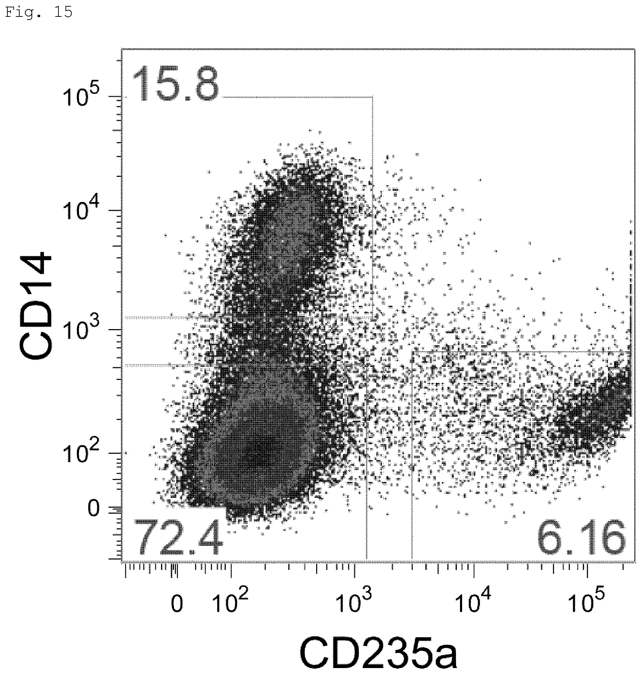

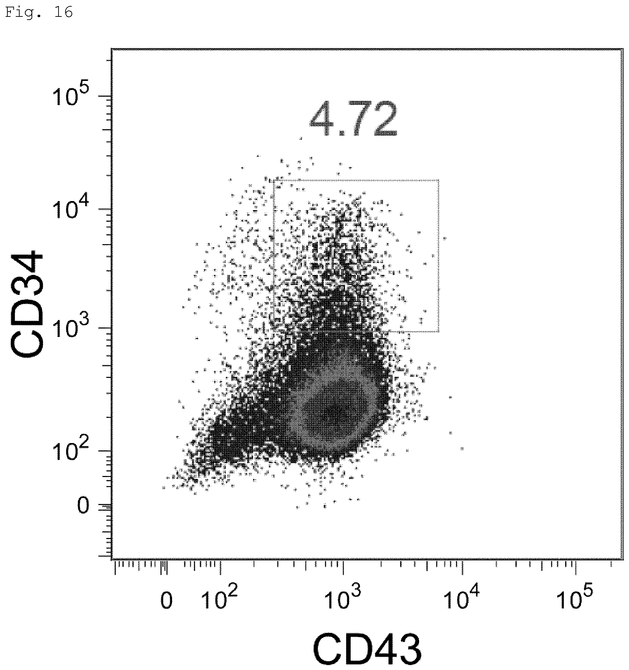

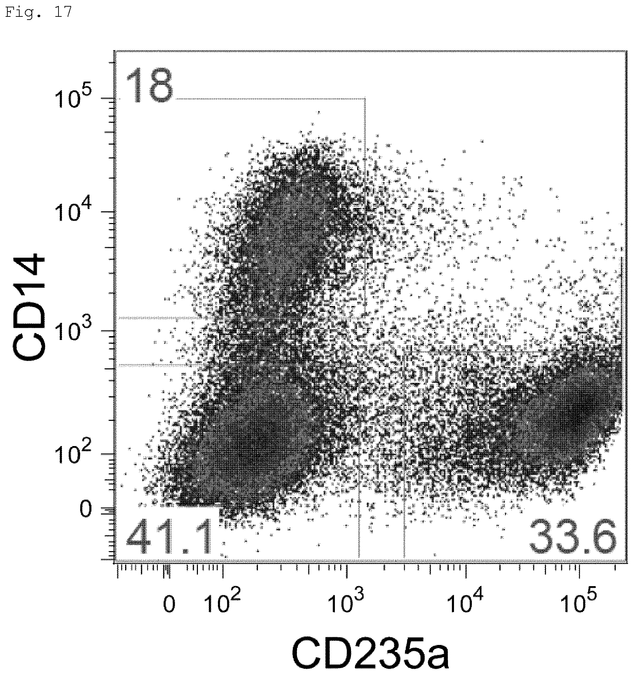

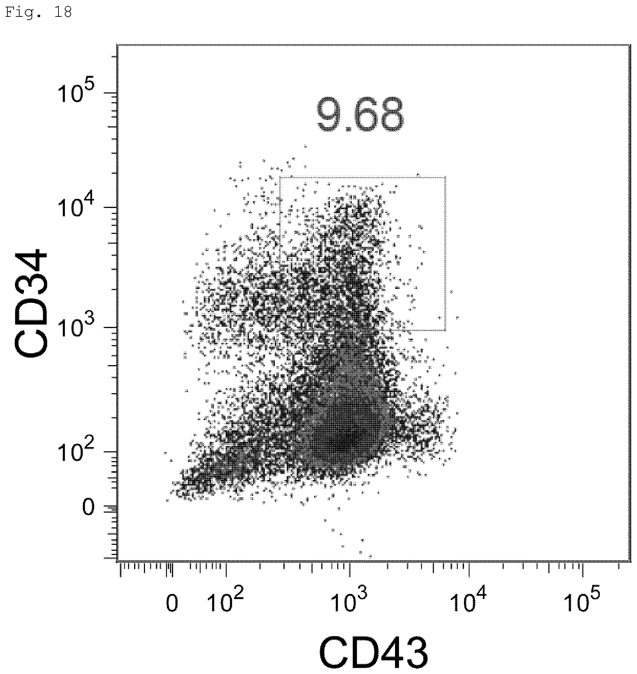

The analysis results are in Figs. 3 and 4 (Example 1; cell count: $5 \times 10^{5}$ cells), Figs. 5 and 6 (Example 1; number of cells: $5 \times 10^{6}$ cells), Fig. 7 and 8 (Example 2; number of cells: $5 \times 10^{5}$ cells), Fig. 9 and 10 (Example 2; number of cells: $5 \times 10^{6}$ cells), Fig. 11 and 12 (Example 3; number of cells: $5 \times 10^{5}$ cells), Fig. 13 and 14 (Example 3; number of cells: $5 \times 10^{6}$ cells), Fig. 15 and 16 (Example 4; number of cells: $5 \times 10^{5}$ cells), and Fig. 17 and 18 (Example 4; number of cells: $5 \times 10^{6}$ cells).

Monocyte lineage cells (CD14+), erythroid cells (CD235a+), etc. were observed.

After differentiation was induced using the culture bag obtained in Example 1, the number of CD34+/CD43+ hematopoietic progenitor cells, which are fractionated using CD34 antibody and CD43 antibody, was determined. When the number of cells on initiation of the culture was $5 \times 10^{5}$-/bag, 10,401 hematopoietic progenitor cells were obtained. When the number of cells on initiation of the culture was $5 \times 10^{6}$-/bag, 3,836 hematopoietic progenitor cells were obtained.

Further, after differentiation was induced using the culture bags obtained in Examples 3 and 4, the number of CD34+/CD43+ hematopoietic progenitor cells, which were fractionated in the same manner as above, was determined. The results are in Table 3.

Table 3

|  | $5 \times 10^{5}$ bag | $5 \times 10^{6}$ bag |
|---|---|---|
| Culture bag of Example 3 | 9,409 cells | 6,479 cells |
| Culture bag of Example 4 | 9,202 cells | 6,425 cells |

These results confirmed that differentiation of T-iPS cells into hematopoietic progenitor cells can be efficiently induced on a large scale regardless of the kind of culture bag used.

When the number of cells on initiation of the culture was $5 \times 10^{5}$-/bag, a higher yield of hematopoietic progenitor cells was obtained as compared with the results obtained with the number of cells on initiation of the culture being $5 \times 10^{6}$-/bag.

(2) Induction of Differentiation to T cells

The differentiation induction ability of CD34+ cells, which were obtained by sorting in the above experiment, into T cells was confirmed by the method disclosed in the above-mentioned known document, Nishimura et al.

The cells obtained from the four bags were collected and mixed. The resulting mixture was transferred to gamma-irradiated OP9-DL1 cells (provided by Riken BioResource Research Center; Watarai H. et al., "Generation of functional NKT cells in vitro from embryonic stem cells bearing rearranged invariant Va14-Ja18 TCRa gene," 2010, Blood, 115, pp. 230-237), and OP9 medium (αMEM medium containing 15% fetal bovine serum, 2 mM L-glutamine, 100 U/ml penicillin, and 100 ng/ml streptomycin) was added. These cells were co-cultured in the presence of FLT-3L (FMS-related tyrosine kinase 3 ligand) and IL-7 (interleukin 7) to induce differentiation of T cell lines. Differentiation tendency was analyzed in the same manner as above using FACS. The culture was performed in an atmosphere containing 5% $CO_2$.

For this analysis, fluorescently labeled antibodies against CD45 (fluorescently labeled with brilliant violet 510), TCRab (fluorescently labeled with FITC), CD3 (fluorescently labeled with APC-Cy7), CD7 (fluorescently labeled with APC), CD5 (fluorescently labeled with PE-Cy7), CD4 (fluorescently labeled with brilliant violet 421), CD8α (fluorescently labeled with PerCP-Cy5.5), and CD8β (fluorescently labeled with PE) were used.

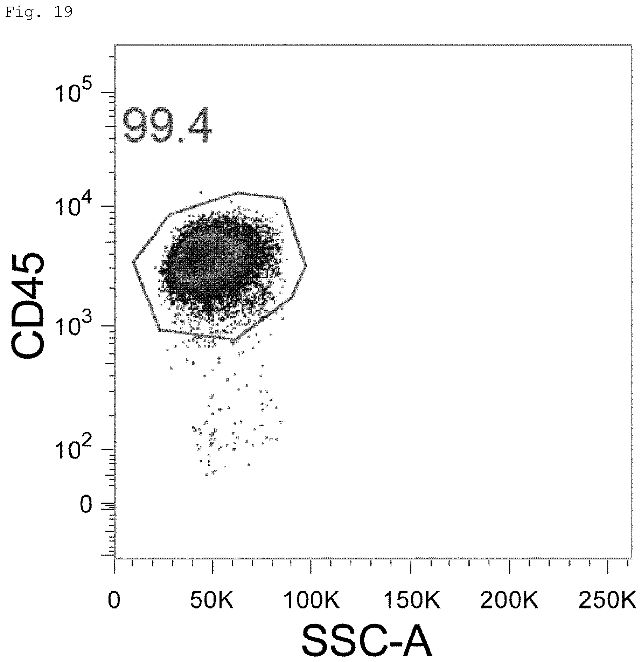

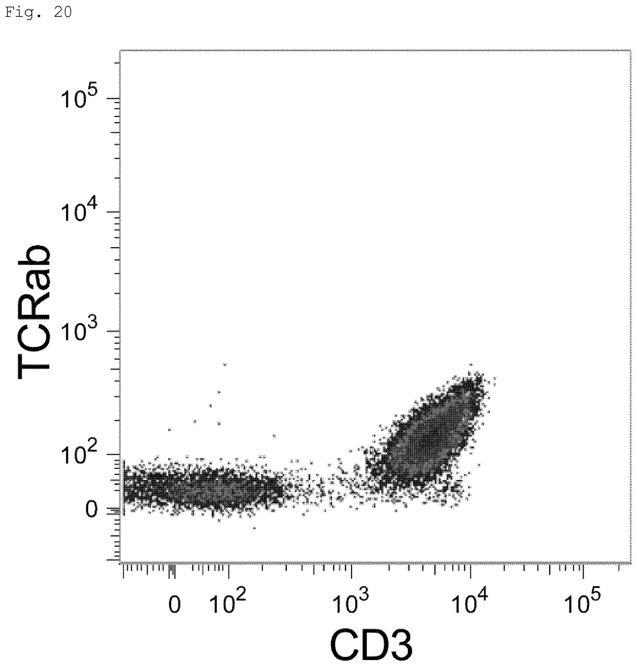

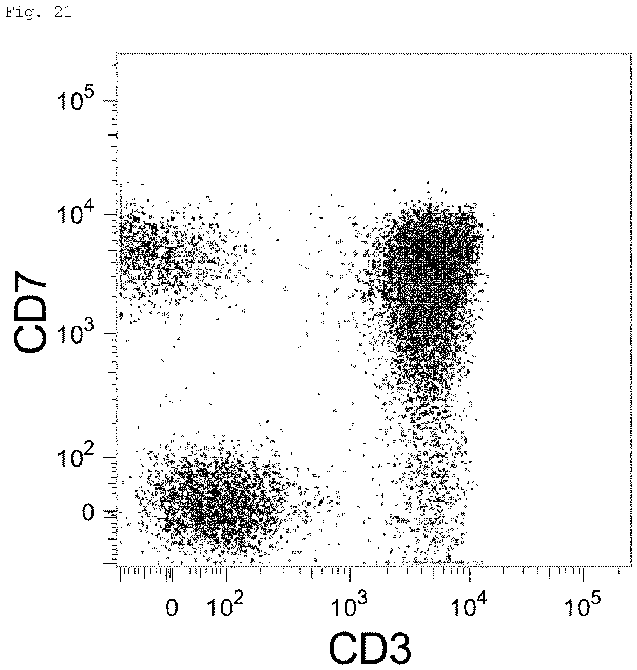

Figs. 19 to 30 show the analysis results.

Figs. 19 to 24 also show the results obtained by analyzing peripheral blood as a control in the same manner. A population of cells that were intermediate in differentiation (CD4+/CD8+) was also confirmed in the population of cells obtained by inducing differentiation of cells obtained from the bag (on day 30)(Figs. 25 to 30). The CD4 expression level and CD8 expression level were each equivalent to the expression level of peripheral blood T cells. This result confirmed that hematopoietic progenitor cells obtained by induction in the bag also had ability to differentiate into T cells with very high efficiency.

Description of the Reference Numerals

1: Culture bag
11: Port
12: Sealing part

**Claims**

1. A method for producing differentiated cells from pluripotent stem cells by an embryoid body (EB) method, comprising the steps of:

   (1) culturing pluripotent stem cells by using a culture bag having a perfluoropolymer on its inner surface to thereby form an embryoid body; and
   (2) inducing differentiation of the pluripotent stem cells contained in the embryoid body obtained in step (1) to thereby obtain differentiated cells.

2. A method for inducing differentiation of pluripotent stem cells by an embryoid body (EB) method, comprising the steps of:

   (1) culturing pluripotent stem cells by using a culture bag having a perfluoropolymer on its inner surface to thereby form an embryoid body; and
   (2) inducing differentiation of the pluripotent stem cells contained in the embryoid body obtained in step (1).

3. The method according to claim 1 or 2, wherein the perfluoropolymer is at least one perfluoropolymer selected from the group consisting of tetrafluoroethylene-hexafluoropropylene copolymers, tetrafluoroethylene-perfluoroalkyl vinyl ether copolymers, and tetrafluoroethylene-hexafluoropropylene-perfluoroalkyl vinyl ether copolymers.

4. The method according to any one of claims 1 to 3, wherein the pluripotent stem cells are induced pluripotent stem cells (iPS cells) or embryonic stem cells (ES cells).

5. The method according to any one of claims 1 to 4, wherein in step (2), the pluripotent stem cells are differentiated into progenitor cells.

6. A culture bag having a perfluoropolymer on its inner surface, the bag being for use in inducing differentiation of pluripotent stem cells by an embryoid body (EB) method.

7. The culture bag according to claim 6, wherein the perfluoropolymer is at least one perfluoropolymer selected from the group consisting of tetrafluoroethylene-hexafluoropropylene copolymers, tetrafluoroethylene-perfluoroalkyl vinyl ether copolymers, and tetrafluoroethylene-hexafluoropropylene-perfluoroalkyl vinyl ether copolymers.

8. The culture bag according to claim 6 or 7, wherein the pluripotent stem cells are induced pluripotent stem cells (iPS cells) or embryonic stem cells (ES cells).

Fig. 1

Fig. 2

Fig. 3

Fig. 4

Fig. 5

Fig. 6

Fig. 7

Fig. 8

Fig. 9

Fig. 10

Fig. 11

Fig. 12

Fig. 13

Fig. 14

Fig. 15

Fig. 16

Fig. 17

Fig. 18

Fig. 19

Fig. 20

Fig. 21

Fig. 22

Fig. 23

Fig. 24

Fig. 25

Fig. 26

Fig. 27

Fig. 28

Fig. 29

Fig. 30

## INTERNATIONAL SEARCH REPORT

| | International application No. |
|---|---|
| | PCT/JP2017/027557 |

A.  CLASSIFICATION OF SUBJECT MATTER
*C12M3/00*(2006.01)i, *C12M1/00*(2006.01)i, *C12N5/071*(2010.01)i, *C12N5/0735*(2010.01)i, *C12N5/10*(2006.01)i, *C12N5/074*(2010.01)n

According to International Patent Classification (IPC) or to both national classification and IPC

B.  FIELDS SEARCHED

Minimum documentation searched (classification system followed by classification symbols)
C12M3/00, C12M1/00, C12N5/071, C12N5/0735, C12N5/10, C12N5/074

Documentation searched other than minimum documentation to the extent that such documents are included in the fields searched
Jitsuyo Shinan Koho          1922–1996    Jitsuyo Shinan Toroku Koho    1996–2017
Kokai Jitsuyo Shinan Koho    1971–2017    Toroku Jitsuyo Shinan Koho    1994–2017

Electronic data base consulted during the international search (name of data base and, where practicable, search terms used)
CAplus/MEDLINE/EMBASE/BIOSIS(STN), JSTPlus/JMEDPlus/JST7580(JDreamIII), PubMed

C.  DOCUMENTS CONSIDERED TO BE RELEVANT

| Category* | Citation of document, with indication, where appropriate, of the relevant passages | Relevant to claim No. |
|---|---|---|
| X<br>Y | WO 2005/037984 A1  (JMS Co., Ltd.),<br>28 April 2005 (28.04.2005),<br>page 2, 2nd paragraph; page 6, 3rd paragraph;<br>page 14, 4th paragraph; page 15, 1st paragraph;<br>page 29, 3rd paragraph to page 33, 3rd<br>paragraph; page 23, 4th paragraph to page 26,<br>3rd paragraph; page 11, 4th paragraph<br>& US 2007/0020754 A1<br>paragraphs [0005], [0026], [0087], [0089],<br>[0175] to [0195], [0135] to [0161], [0066]<br>& US 2010/0137811 A1    & EP 1679365 A1 | 6–8<br>1–5 |

☒  Further documents are listed in the continuation of Box C.          ☐  See patent family annex.

| | |
|---|---|
| *     Special categories of cited documents: | "T"   later document published after the international filing date or priority date and not in conflict with the application but cited to understand the principle or theory underlying the invention |
| "A"   document defining the general state of the art which is not considered    to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
|---|---|
| 17 October 2017 (17.10.17) | 24 October 2017 (24.10.17) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

**INTERNATIONAL SEARCH REPORT**

| | International application No. |
|---|---|
| | PCT/JP2017/027557 |

C (Continuation).   DOCUMENTS CONSIDERED TO BE RELEVANT

| Category* | Citation of document, with indication, where appropriate, of the relevant passages | Relevant to claim No. |
|---|---|---|
| X<br>Y | JP 63-198972 A  (Daikin Industries, Ltd.),<br>17 August 1988 (17.08.1988),<br>claims 2, 3; example 1; fig. 1; page 383, left<br>column, 6th paragraph to right column, 1st<br>paragraph<br>(Family: none) | 6-8<br>1-5 |
| X<br>Y | JP 2014-239742 A  (Daikin Industries, Ltd.),<br>25 December 2014 (25.12.2014),<br>claim 1; paragraphs [0022], [0068]<br>(Family: none) | 6-8<br>1-5 |
| Y | JP 2015-519890 A  (Kaufman Dan S. et al.),<br>16 July 2015 (16.07.2015),<br>example 1<br>& US 2013/0287751 A1<br>example 1<br>& WO 2013/163171 A1    & EP 2841563 A1 | 1-8 |
| Y | JP 2012-519005 A  (Cellular Dynamics<br>International, Inc.),<br>23 August 2012 (23.08.2012),<br>examples 2 to 4<br>& US 2010/0279403 A1<br>examples 2 to 4<br>& US 2013/0210141 A1    & WO 2010/099539 A1<br>& EP 2401364 A1 | 1-8 |
| Y | WO 2014/200030 A1  (Kyoto University),<br>18 December 2014 (18.12.2014),<br>examples 2, 4<br>& US 2016/0137981 A1<br>examples 2, 4 | 1-8 |
| A | JP 2016-520307 A  (Corning Inc.),<br>14 July 2016 (14.07.2016),<br>entire text<br>& US 2014/0322806 A1<br>entire text<br>& WO 2014/179196 A1    & EP 2992079 A1 | 1-8 |
| A | NISHIMURA, T., et al., Generation of<br>rejuvenated antigen-specific T cells by<br>reprogramming to pluripotency and<br>redifferentiation, 2013, Cell Stem Cell, Vol.12,<br>p.114-126 entire text | 1-8 |

Form PCT/ISA/210 (continuation of second sheet) (January 2015)

## REFERENCES CITED IN THE DESCRIPTION

**Patent documents cited in the description**

- WO 2005001019 A

**Non-patent literature cited in the description**

- **NISHIMURA et al.** Generation of Rejuvenated Antigen-Specific T Cells by Reprogramming to Pluripotency and Redifferentiation. *Cell Stem Cell,* 2013, vol. 12, 114-126

- Generation of functional NKT cells in vitro from embryonic stem cells bearing rearranged invariant Va14-Ja18 TCRa gene. **WATARAI H. et al.** Blood. Riken BioResource Research Center, 2010, vol. 115, 230-237